# EUROPEAN PATENT APPLICATION

(11) **EP 4 659 681 A1**
(43) Date of publication of application: **10.12.2025**
(21) Application number: 25179744.5
(22) Date of filing: 29.05.2025
(51) Int. Cl.: A61B 6/08, A61B 6/00, A61B 6/58

(54) **RADIOGRAPHY SYSTEM AND IMAGING UNIT**

(30) Priority: 06.06.2024 JP 2024092508
(71) Applicant: FUJI-FILM Corporation, Minato-ku Tokyo 106-8620 (JP)
(72) Inventor: HORIUCHI, Hisatsugu, Kanagawa (JP); MATSUURA, Masayoshi, Kanagawa (JP); TANINAI, Koji, Kanagawa (JP); NISHINO, Naoyuki, Kanagawa (JP); KOBAYASHI, Takeyasu, Kanagawa (JP)
(74) Representative: Dehns Germany Partnerschaft mbB

(57) **Abstract**

A radiography system (10) used for radiography includes two imaging units (11) including an imaging unit (11S) including a radiation source (12) and an imaging unit (11D) including a radiation detector (13), in which at least one imaging unit of the two imaging units (11) includes a projection apparatus (21) that projects a positioning indicator used for relative positioning between the two imaging units (11).

## Description

### BACKGROUND OF THE INVENTION

### 1. Field of the Invention

The present disclosure relates to a radiography system and an imaging unit.

### 2. Description of the Related Art

JP2016-150155A and JP2014-533549A disclose an X-ray imaging system that includes an X-ray source and an X-ray detector and images a subject using X-rays, as an example of a radiography system. JP2016-150155A and JP2014-533549A also include a projection apparatus that projects positioning information used for positioning the subject with respect to the X-ray detector.

### SUMMARY OF THE INVENTION

In addition to positioning of the subject, relative positioning between the X-ray source and an imaging table including the X-ray detector is also important in X-ray imaging. Examples of such positioning include appropriately setting a source to image receptor distance (SID) that is a spacing between the X-ray source and the imaging table. As is well known, there is a ceiling-suspended X-ray source suspended from a ceiling traveling device. In the case of such an X-ray source, positioning such as appropriately setting the SID can be relatively simply performed by moving the X-ray source along a rail of the ceiling traveling device.

There is also a floor-traveling X-ray source including a traveling mechanism for traveling on a floor. However, such an X-ray source poses a problem in that appropriate positioning between the X-ray source and the imaging table is difficult to achieve because a degree of freedom of a moving range is higher than that of the ceiling-suspended X-ray source of which the moving range is restricted by the rail. Not only the X-ray source but also a floor-traveling imaging table including a traveling mechanism for traveling on the floor have been developed. In the case of using the floor-traveling X-ray source and the floor-traveling imaging table in combination, the degree of freedom of the moving range is further increased, and this poses a problem in that appropriate positioning is more difficult to achieve.

Positioning between both may be difficult depending on aspects of the X-ray source and the X-ray detector, and a technique for facilitating such positioning is desired.

The disclosed technology provides a radiography system and an imaging unit that facilitate relative positioning between a radiation source and a radiation detector compared to that in the related art.

According to an aspect of the disclosed technology, there is provided a radiography system used for radiography, comprising two imaging units including an imaging unit including a radiation source and an imaging unit including a radiation detector, in which at least one imaging unit of the two imaging units includes a projection apparatus that projects a positioning indicator used for relative positioning between the two imaging units.

In an embodiment, at least one imaging unit of the two imaging units may include an optical sensor that optically detects the positioning indicator, and a processor configured to determine whether or not a relative positional relationship between the two imaging units is appropriate based on the positioning indicator detected by the optical sensor may be provided.

In an embodiment, in a case where at least one imaging unit of the two imaging units includes the projection apparatus and the other imaging unit includes the optical sensor and the processor, the processor included in the other imaging unit may be configured to determine whether or not the relative positional relationship is appropriate by determining whether or not a host imaging unit which is the other imaging unit is present at a target position represented by the positioning indicator that is projected by the projection apparatus of a counterpart imaging unit which is the one imaging unit and that is detected by the optical sensor.

In an embodiment, in a case where at least one imaging unit of the two imaging units includes the optical sensor and the processor in addition to the projection apparatus, the processor included in the one imaging unit may be configured to determine whether or not the relative positional relationship is appropriate by determining whether or not a counterpart imaging unit which is the other imaging unit is present at a target position represented by the positioning indicator that is projected by the projection apparatus of a host imaging unit which is the one imaging unit and that is detected by the optical sensor.

In an embodiment, each of the two imaging units may include the optical sensor and the processor in addition to the projection apparatus.

In an embodiment, at least the imaging unit including the radiation detector out of the two imaging units may include the projection apparatus.

In an embodiment, at least one imaging unit of the two imaging units may include a traveling mechanism that travels on a floor.

In an embodiment, the traveling mechanism may be an electric traveling mechanism.

In an embodiment, the imaging unit that is at least one of the two imaging units and that includes the optical sensor and the processor may include an electric traveling mechanism that electrically travels on a floor, and the processor may be configured to automatically perform registration by controlling the electric traveling mechanism based on a determination result related to the relative positional relationship.

In an embodiment, the imaging unit that is at least one of the two imaging units and that includes the projection apparatus may include an engaging mechanism that engages with a location set in advance, the other imaging unit in which the processor is configured to determine whether or not the relative positional relationship is appropriate based on the positioning indicator projected by the projection apparatus may include an electric traveling mechanism that electrically travels on a floor, and the electric traveling mechanism may move to a target position represented by the positioning indicator based on a determination result of the processor.

In an embodiment, the processor may be provided in an apparatus different from the two imaging units.

In an embodiment, the different apparatus may be a console for operating at least one of the two imaging units.

In an embodiment, the projection apparatus may determine a projection position of the positioning indicator in accordance with a designated imaging menu.

In an embodiment, the projection apparatus may project a positioning indicator representing a plurality of target positions as the positioning indicator.

In an embodiment, the imaging unit including the radiation detector may be a radiation detection panel having a portable housing, and the projection apparatus may project a line-shaped marker to an outside of the housing as the positioning indicator.

In an embodiment, at least one imaging unit of the two imaging units may include a projection apparatus that projects a region indicator indicating a region requiring entry with caution in addition to the positioning indicator.

According to another aspect of the disclosed technology, there is provided an imaging unit that is used for radiography and that is one of two imaging units including an imaging unit including a radiation source and an imaging unit including a radiation detector, the imaging unit comprising a projection apparatus that projects a positioning indicator used for relative positioning between the two imaging units.

According to the disclosed technology, relative positioning between a radiation source and a radiation detector can be facilitated compared to that in the related art.

### BRIEF DESCRIPTION OF THE DRAWINGS

Fig. 1 is a diagram illustrating an example of a schematic configuration of a radiography system.
Fig. 2 is a block diagram illustrating an example of a functional configuration of an imaging unit.
Fig. 3 is a diagram illustrating a configuration example of changing a projection position in accordance with an imaging menu.
Fig. 4 is a block diagram illustrating an example of the projection position.
Fig. 5 is a diagram illustrating an example of a processing procedure of position determination.
Fig. 6 is a diagram illustrating an example of an electric traveling mechanism.
Fig. 7 is a diagram illustrating an example of a controller of the electric traveling mechanism.
Fig. 8 is a diagram illustrating an example of a processing procedure of automatic positioning.
Fig. 9 is a diagram illustrating an example of combining the automatic positioning with an engaging mechanism.
Fig. 10 is a diagram illustrating an example of a pattern of a combination of a projection apparatus and a camera.
Fig. 11 is a diagram illustrating another example of the pattern of the combination of the projection apparatus and the camera.
Fig. 12 is a diagram illustrating an example of a processing procedure of the example in Fig. 11.
Fig. 13 is a diagram illustrating still another example of the pattern of the combination of the projection apparatus and the camera.
Fig. 14 is a diagram illustrating a list of patterns of the combination of the projection apparatus and the camera.
Fig. 15 is a diagram illustrating an example of a marker including a plurality of target positions.
Fig. 16 is a diagram illustrating another example of the marker including the plurality of target positions.
Fig. 17 is a diagram illustrating an example of providing the projection apparatus in a portable detection panel.
Fig. 18 is a diagram illustrating an example of a region indicator requiring entry with caution.
Fig. 19 is a diagram illustrating an example of a marker other than the marker for positioning.
Fig. 20 is a diagram illustrating another example of the marker other than the marker for positioning.
Fig. 21 is a diagram illustrating an example of providing a processor in a console.

### DESCRIPTION OF THE PREFERRED EMBODIMENTS

### First Embodiment

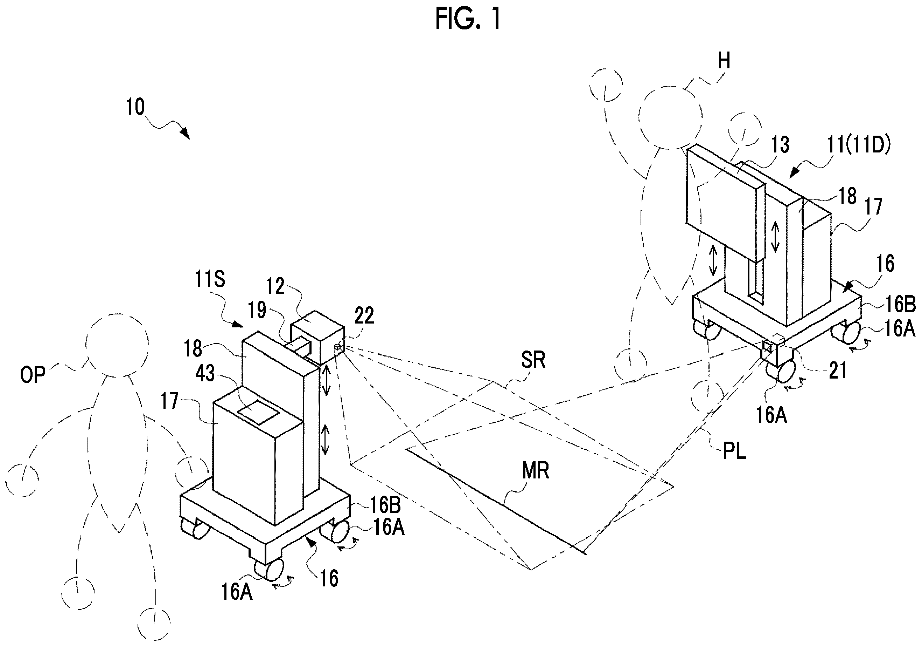

Fig. 1 is a schematic diagram illustrating an example of a configuration of a radiography system 10 that performs radiography of a subject H. The radiography system 10 comprises a radiation source unit 11S and a panel unit 11D. For example, these units are installed in a radiography room and are operated by an operator OP such as a medical radiologist. The radiation source unit 11S includes an emission portion 12. The emission portion 12 is an example of a "radiation source" according to the disclosed technology. The emission portion 12 includes an X-ray tube that generates X-rays which are an example of radiation, an emission field limiter that limits an emission range of the X-rays, and the like. The radiation source unit 11S is also provided with a high-voltage generator that generates a high voltage to be supplied to the X-ray tube, and the like.

The panel unit 11D includes a detection panel 13 that is an example of a radiation detection panel. For example, the detection panel 13 is a flat panel detector having a detection surface on which pixels that detect X-rays and output electric signals corresponding to an incidence amount of the X-rays are two-dimensionally arranged. The detection panel 13 is an example of a "radiation detector" according to the disclosed technology.

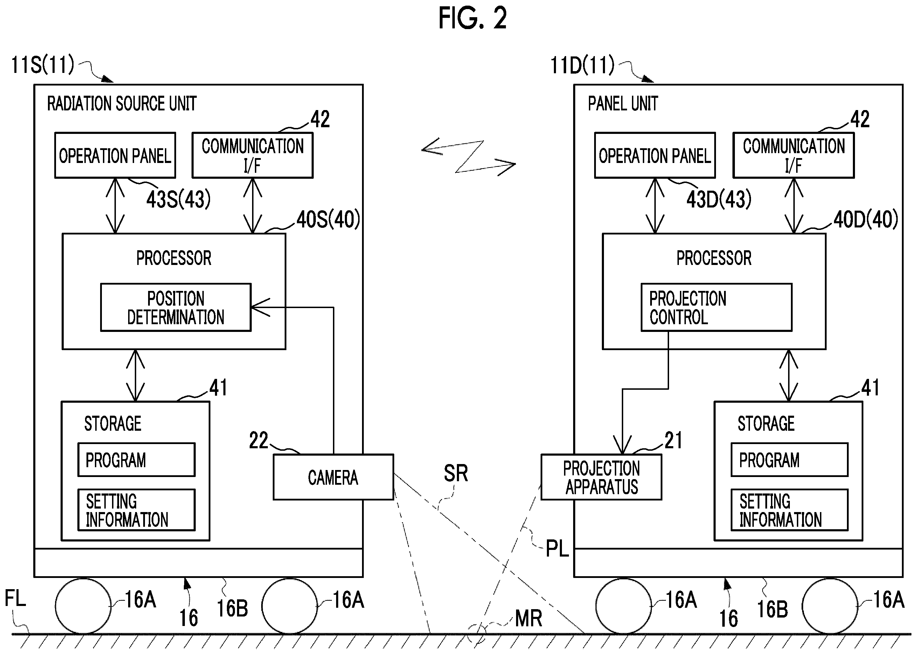

The radiography system 10 captures an X-ray image of the subject H by emitting X-rays from the emission portion 12 and detecting the X-rays transmitted through the subject H via the detection panel 13. Each of the radiation source unit 11S and the panel unit 11D includes a carriage portion 16 including wheels 16A. For example, the carriage portion 16 includes a base portion 16B that is a body part having a rectangular planar shape. The wheels 16A are provided at four corners of the base portion 16B, and the carriage portion 16 is of a four-wheel type. For example, each wheel 16A is a revolving wheel that revolves about a revolution axis extending in a height direction (referred to as a vertical direction) orthogonal to a rotation axis in rotating to travel. The carriage portion 16 can be manually caused to travel on a floor FL (refer to Fig. 2 and the like). Thus, the operator OP can move installation locations of the radiation source unit 11S and the panel unit 11D by manually causing the carriage portion 16 to travel. The carriage portion 16 is an example of a "traveling mechanism" that travels on the floor FL.

The radiation source unit 11S includes a body part 17 and a movable mechanism for changing a height of the emission portion 12. The movable mechanism is composed of a movable portion 18 of which a height changes with respect to the body part 17, and an arm 19 of which a height changes with respect to the movable portion 18. The emission portion 12 is provided at a tip end of the arm 19. The emission portion 12 is provided at a free end of the arm 19. While illustration is not provided, the arm 19 is provided with a swing mechanism that changes an emission direction by tilting the emission portion 12. The body part 17 incorporates various electric components, and an operation panel 43 is provided on its upper surface.

Like the radiation source unit 11S, the panel unit 11D also includes a body part 17 and a movable mechanism that changes a height of the detection panel 13. The movable mechanism of the panel unit 11D is also composed of a movable portion 18 of which a height changes with respect to the body part 17, and an arm (not illustrated) of which a height with respect to the movable portion 18 changes, and is the same as the movable mechanism of the radiation source unit 11S. The detection panel 13 is provided at a free end of the arm. Like the body part 17 of the radiation source unit 11S, the body part 17 also incorporates various electric components, and the operation panel 43 is provided on its upper surface.

While the body parts 17, the movable portions 18, and the arms 19 in each of the radiation source unit 11S and the panel unit 11D are parts having different incorporated components, shapes, and the like, differences between both are not important in the disclosed technology. Thus, both of them will be designated by the same reference numerals. Both of the radiation source unit 11S and the panel unit 11D are examples of an "imaging unit" according to the disclosed technology. Hereinafter, both of the radiation source unit 11S and the panel unit 11D will be referred to as imaging units 11 unless distinction therebetween is required.

As illustrated in Fig. 1, for example, in imaging the subject H in a standing posture, the radiation source unit 11S and the panel unit 11D are disposed to face each other with the subject H interposed therebetween.

In radiography, in addition to positioning the subject H with respect to the detection panel 13, relative positioning between the radiation source unit 11S and the panel unit 11D is performed such that the X-rays emitted from the emission portion 12 are appropriately incident on the detection panel 13 with which the subject H is positioned. The radiography system 10 comprises a projection apparatus 21 and a camera 22 as an apparatus for supporting relative positioning between the radiation source unit 11S and the panel unit 11D. In the present example, the projection apparatus 21 is provided in the panel unit 11D, and the camera 22 is provided in the radiation source unit 11S.

The projection apparatus 21 projects a marker MR to the floor FL by emitting projection light PL. The marker MR is a positioning indicator used for relative positioning between two imaging units 11 including the radiation source unit 11S and the panel unit 11D and is an example of a "positioning indicator" according to the disclosed technology. For example, the marker MR has a line shape and indicates a target position of the radiation source unit 11S to be positioned with respect to the panel unit 11D. By aligning the radiation source unit 11S with a position of the marker MR, a source-to-image distance (SID) that is a spacing between the radiation source unit 11S and the panel unit 11D can be appropriately secured.

The projection apparatus 21 includes, for example, a light source that emits light, an optical modulator that generates the projection light PL corresponding to a shape of the marker MR which is a projection image, and a projection optical system that projects the generated projection light PL to the floor FL. For example, the light source is a light emitting diode (LED) or a laser diode (LD). Examples of the optical modulator include a liquid crystal display (LCD) and a digital micromirror device (DMD). Since the marker MR of the present example has a line shape, a line laser projector composed of, for example, an LD and a projection optical system may be used as the projection apparatus 21 without using the optical modulator.

A plurality of target positions for positioning the radiation source unit 11S are present depending on an imaging menu (described later). Thus, the projection apparatus 21 includes a mechanism for changing a projection position of the marker MR. As a method of changing the projection position of the marker MR, not only a method of changing the position of the marker MR in a projection region through image processing but also a method of changing the projection position of the marker MR by changing a position, a direction, a focal length, and the like of the projection optical system are considered.

For example, the projection apparatus 21 is provided in the carriage portion 16. While various positions for providing the projection apparatus 21 are considered, a position at which the projection apparatus 21 does not cast a shadow on the subject H is preferred. The projection apparatus 21 can also be provided in the detection panel 13 or the movable portion 18. In this case, it is preferable to provide a mechanism for adjusting the projection position in accordance with displacement of the detection panel 13 or the movable portion 18 such that the projection position of the marker MR is projected to a desired position even in a case where the detection panel 13 or the movable portion 18 is displaced.

For example, the camera 22 is an optical camera. As is well known, the camera 22 includes an image sensor that outputs an electric signal corresponding to a received light quantity, such as a complementary metal-oxide-semiconductor (CMOS) image sensor or a charge coupled device (CCD) image sensor, and an imaging optical system that forms an image of a subject on an imaging surface of the image sensor. The camera 22 functions as a sensor that optically detects the marker MR by imaging the marker MR as an imaging target. The camera 22 is an example of an "optical sensor" that optically detects the positioning indicator according to the disclosed technology.

The camera 22 acquires an image of the imaging target within an imaging range SR determined in accordance with an angle of view of the imaging optical system and a size of the image sensor. The image of the imaging range SR captured by the camera 22 is output to a processor 40S (described later; refer to Fig. 2) and is used for determining whether or not relative positioning between the two imaging units 11 including the radiation source unit 11S and the panel unit 11D is appropriate.

For example, the camera 22 is provided in the emission portion 12. While various positions for providing the camera 22 are considered, a position at which the camera 22 does not cast a shadow on the subject H is preferred, like the projection apparatus 21. The camera 22 may also be provided in the carriage portion 16. In a case where the camera 22 is provided in a movable portion such as the emission portion 12 as in the present example, a position of the imaging range SR also changes in accordance with displacement of the movable portion. Thus, in performing position determination of the imaging unit 11 based on the marker MR, for example, a position of the emission portion 12 is set to a reference position for the position determination such that the position of the camera 22 remains constant.

Fig. 2 is a block diagram illustrating an electric configuration of the imaging unit 11. The panel unit 11D that is an example of the imaging unit 11 comprises a processor 40D, a storage 41, a communication interface (I/F) 42, and an operation panel 43D. The panel unit 11D also comprises a battery (not illustrated) that supplies power to each part.

The processor 40D not only controls the whole panel unit 11D in an integrated manner but also functions as a controller that controls the detection panel 13, and a controller that controls the projection apparatus 21. For example, the processor 40D is composed of a central processing unit (CPU) and a memory such as a random access memory (RAM) and functions as various controllers by executing a program loaded into the memory.

The storage 41 is a data storage that is composed of a hard disk drive, a solid state drive, a non-volatile memory, and the like and stores not only the program but also data of various types of setting information.

The communication I/F 42 is, for example, a wireless communication portion and executes wireless communication with another imaging unit 11 such as the radiation source unit 11S.

The operation panel 43D is an operator for inputting an operation instruction and is composed of, for example, a touch panel display.

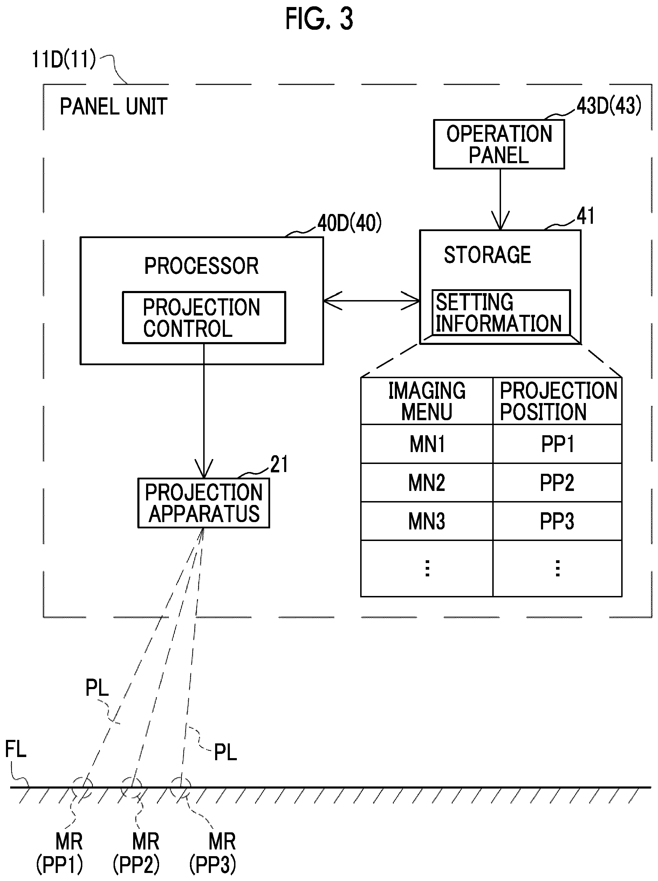

As illustrated in Fig. 3, a correspondence relationship between the imaging menu (for example, MN1, MN2, and MN3) and the projection position (PP1, PP2, PP3, and the like) of the marker MR is recorded in the setting information of the storage 41 of the panel unit 11D. The imaging menu defines an imaging technique including a part to be imaged, an emission direction of the X-rays, and the like. The SID may be changed in accordance with the imaging menu. Imaging may be performed in a state where the subject H is placed on a wheelchair. In this case, a long SID is required.

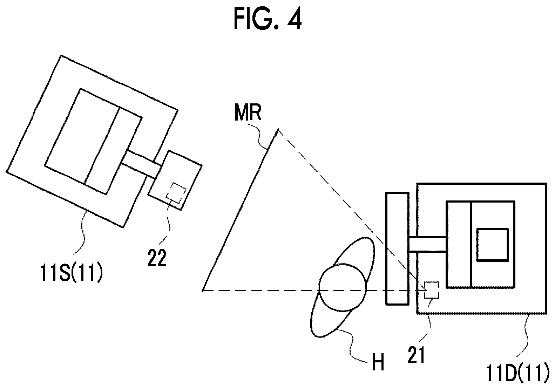

As illustrated in Fig. 4, for example, in an imaging technique for imaging a joint or the like of the subject H, the subject H may be obliquely disposed with respect to the detection panel 13, and the emission direction of the X-rays may also be oblique with respect to the detection panel 13. In this case, a direction of the radiation source unit 11S may also be oblique with respect to the panel unit 11D. Thus, the line of the marker MR for relatively positioning the radiation source unit 11S may also have to be obliquely projected.

The correspondence relationship between the imaging menu and the projection position of the marker MR is recorded in the setting information. For example, the imaging menu is designated by the operator OP through the operation panel 43D. In controlling projection, the processor 40D determines the projection position of the marker MR in accordance with the designated imaging menu. The processor 40D controls the projection apparatus 21 such that the marker MR is projected to the determined projection position.

With reference to Fig. 2, the radiation source unit 11S also comprises the processor 40S, a storage 41, a communication interface (I/F) 42, and an operation panel 43, like the panel unit 11D. The radiation source unit 11S also comprises a battery (not illustrated) that supplies power to each part.

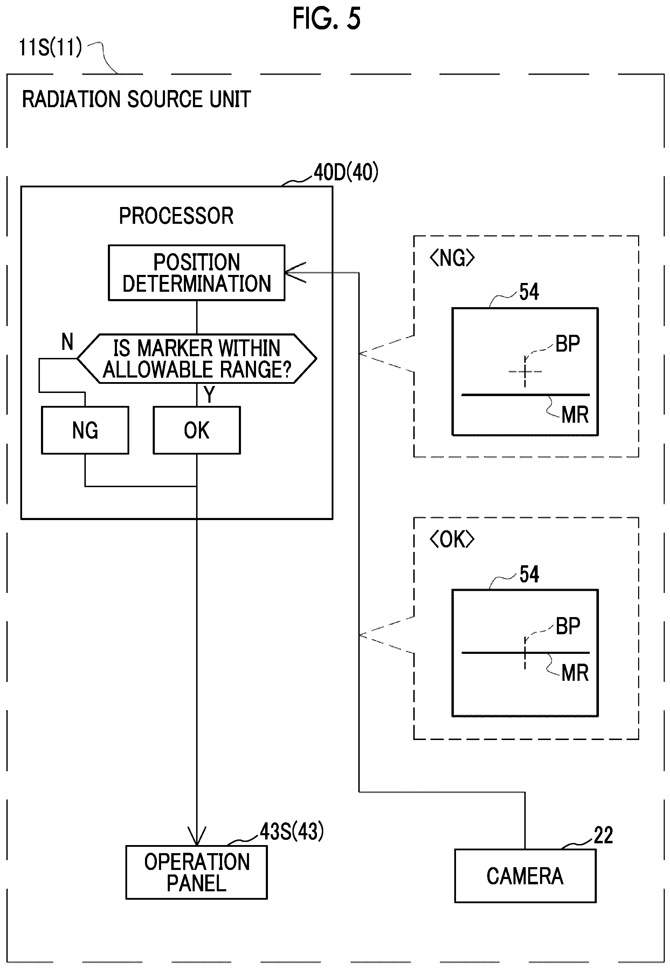

The processor 40S not only controls the whole radiation source unit 11S in an integrated manner but also functions as a controller that controls emission of the X-rays of the emission portion 12, and a controller that controls the camera 22. The processor 40S performs the position determination based on an image 54 (refer to Fig. 5) acquired from the camera 22. For example, the processor 40S is composed of a central processing unit (CPU) and a memory such as a random access memory (RAM) and functions as various controllers by executing a program loaded into the memory.

The storage 41 is a data storage that is composed of a hard disk drive, a solid state drive, a non-volatile memory, and the like and stores not only the program but also data of various types of setting information.

The communication I/F 42 is, for example, a wireless communication portion and executes wireless communication with another imaging unit 11 such as the panel unit 11D.

The operation panel 43S is an operator for inputting an operation instruction and is composed of, for example, a touch panel display.

Like the imaging unit 11, processors 40 and operation panels 43 will be distinguished using subdivision reference numerals S and D in a case where distinction between the processor 40S and the operation panel 43S of the radiation source unit 11S and the processor 40D and the operation panel 43D of the panel unit 11D is required. Both of the processors 40S and 40D and both of the operation panels 43S and 43D will be referred to as the processors 40 and the operation panels 43 unless distinction therebetween is required.

As illustrated in Fig. 5, the processor 40S determines whether or not a relative positional relationship with respect to the panel unit 11D is appropriate based on the marker MR included in the image 54 acquired from the camera 22. More specifically, the following is performed. That is, in the present example, in a case where the radiation source unit 11S provided with the processor 40S is referred to as the host imaging unit 11, the marker MR is a marker projected from the projection apparatus 21 of the panel unit 11D that is the counterpart imaging unit 11 of registration. Thus, the processor 40S of the radiation source unit 11S determines whether or not the host imaging unit 11 (in the present example, the radiation source unit 11S) is present at the target position represented by the marker MR projected from the counterpart imaging unit 11 (in the present example, the panel unit 11D).

In a case where the marker MR is within an allowable range based on the image 54, the processor 40S determines that the relative positional relationship is appropriate (indicated by OK). In a case where the marker MR is out of the allowable range, the processor 40S determines that the relative positional relationship is inappropriate (indicated by NG). For example, whether or not the marker MR is within the allowable range is determined based on a ratio of match between a reference position BP set in advance, such as a center position in the image 54, and the position of the marker MR. For example, as illustrated in Fig. 5, in a case where error in the position of the marker MR with respect to the reference position BP is greater than or equal to a distance set in advance, a determination of "NG" is made. In a case where the error is within the distance set in advance, a determination of "OK" is made.

In determining whether or not the marker MR is within the allowable range, the processor 40S also considers a posture of the marker MR with respect to the reference position BP in addition to a distance between the marker MR and the reference position BP. As illustrated in Fig. 5, for example, the reference position BP has a cross shape, and the posture of the line-shaped marker MR with respect to the reference position BP can also be determined. The processor 40 determines whether or not the relative positional relationship is appropriate by determining whether or not the posture of the marker MR is within an allowable range set in advance.

The processor 40S performs such determination and outputs a determination result to the operation panel 43S. The operator OP adjusts a position of the radiation source unit 11S while checking the determination result and manually moving the radiation source unit 11S. In addition to the determination results of "OK" and "NG", an image showing the ratio of match between the marker MR and the reference position BP is preferably displayed in real time on the operation panel 43S, like the image 54 illustrated in Fig. 5. Accordingly, the operator OP can check in which direction the radiation source unit 11S is to be moved to match the target position.

As described above, the radiography system 10 according to the disclosed technology is used for radiography and includes two imaging units including the imaging unit 11 (for example, the radiation source unit 11S) including the radiation source (for example, the emission portion 12) and the imaging unit 11 (for example, the panel unit 11D) including the radiation detector (for example, the detection panel 13). At least one imaging unit 11 (in the above example, the panel unit 11D) of the two imaging units 11 comprises the projection apparatus 21 that projects the positioning indicator (for example, the marker MR) used for relative positioning between the two imaging units 11.

Since two imaging units 11 are used in combination during radiography, the relative positional relationship between the two imaging units 11 is important. Relative positioning between the two imaging units 11 is easily performed by projecting the positioning indicator used for relative positioning from one imaging unit 11.

In the first embodiment, at least one imaging unit 11 of the two imaging units 11 includes the traveling mechanism (for example, the carriage portion 16) that travels on the floor. The imaging unit 11 including the traveling mechanism has a higher degree of freedom in a moving range than an imaging unit attached to a ceiling traveling device or the like. As the degree of freedom in the moving range is higher, accurate positioning is more difficult to perform. Thus, the disclosed technology that facilitates positioning by projecting the positioning indicator is effective in a case where the imaging unit 11 includes the traveling mechanism.

The disclosed technology is more effective in the first embodiment because both of the two imaging units 11 include the traveling mechanism, and both of the two imaging units 11 have a high degree of freedom in the moving range.

In the radiography system 10 according to the first embodiment, at least one imaging unit 11 (in the above example, the radiation source unit 11S) of the two imaging units 11 comprises the optical sensor (for example, the camera 22) that optically detects the positioning indicator (for example, the marker MR), and includes the processor 40 (in the above example, the processor 40S) that determines whether or not the relative positional relationship between the two imaging units 11 is appropriate based on the positioning indicator detected by the optical sensor. Thus, simple and high-accuracy positioning can be performed compared to positioning performed by the operator OP while visually checking the position of the marker MR.

While the above example describes the camera 22 as an example of the optical sensor, the optical sensor may not be a sensor that detects the marker MR as an image like the camera 22 and may detect the marker MR based on intensity of light of the marker MR like a simple photo sensor.

In the first embodiment, at least one imaging unit 11 (in the above example, the panel unit 11D) of the two imaging units 11 includes the projection apparatus 21, and the other imaging unit 11 (in the above example, the radiation source unit 11S) includes the optical sensor (for example, the camera 22) and the processor 40 (in the above example, the processor 40S). In this case, the processor 40S included in the radiation source unit 11S, which is the other imaging unit 11, performs the following determination. That is, the processor 40S determines whether or not the relative positional relationship is appropriate by determining whether or not the host radiation source unit 11S, which is the other imaging unit 11, is present at the target position represented by the marker MR that is the positioning indicator projected by the projection apparatus 21 of the counterpart panel unit 11D, which is the one imaging unit 11, and detected by the optical sensor.

This means that the processor 40S that determines the relative positional relationship is provided in the radiation source unit 11S that is the imaging unit 11 moved in accordance with the target position of the marker MR. Thus, for example, the operator OP can move the radiation source unit 11S while checking the determination result displayed on the operation panel 43S. Accordingly, high usability is achieved.

In the first embodiment, the imaging unit 11 (for example, the panel unit 11D) including the radiation detector out of the two imaging units 11 includes the projection apparatus 21. In positioning the two imaging units 11 during radiography, it is general to position the radiation source unit 11S with respect to the panel unit 11D after the subject H and the detection panel 13 are positioned. Thus, the configuration as in the first embodiment conforms to the actual workflow of radiography and provides high usability.

In the first embodiment, as illustrated in Fig. 3, the projection apparatus 21 determines the projection position of the positioning indicator (for example, the marker MR) in accordance with the designated imaging menu. This is effective in a case where the target position of positioning varies depending on the imaging menu.

### Second Embodiment

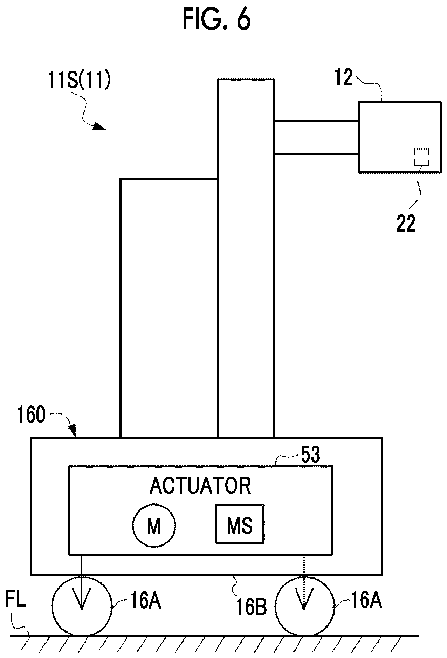

While the carriage portions 16 of the two imaging units 11 are manual traveling mechanisms in the first embodiment, the traveling mechanisms may be electric traveling mechanisms as in a second embodiment illustrated in Fig. 6. A carriage portion 160 is an example of an "electric traveling mechanism" according to the disclosed technology. The imaging unit 11 illustrated in Fig. 6 is the radiation source unit 11S, and an example of providing the electric traveling mechanism in the radiation source unit 11S will be described.

The carriage portion 160 incorporates an electric circuit for electric traveling and electric components such as an actuator 53. For example, the carriage portion 160 is of a four-wheel type including four wheels 16A, and each wheel 16A is a revolving wheel. These points indicate that the carriage portion 160 is the same as the carriage portion 16. For example, all of the wheels 16A are drive wheels that are electrically rotated. Two of the four wheels 16A may be steering wheels, and the remaining two may be drive wheels.

The actuator 53 includes a motor M that drives the wheels 16A. The motor M includes a motor for causing the wheels 16A to rotate to travel and a motor for causing the wheels 16A to revolve. A measurement sensor MS measures rotation amounts, rotation directions, revolution directions, revolution angles, and the like of the wheels 16A and a moving direction, a moving amount, and the like of the carriage portion 160. Measurement values measured by the measurement sensor MS are input into the processor 40S. For example, the measurement sensor MS is composed of a rotary encoder and an acceleration sensor. The processor 40S can execute a traveling control such as self position estimation of the carriage portion 16 based on the received measurement values.

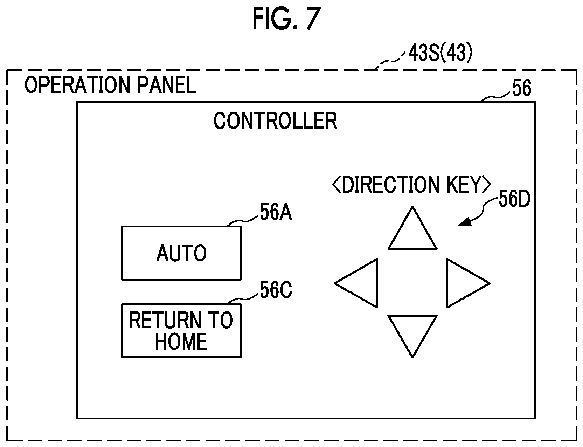

Fig. 7 is an example of a screen, displayed on the operation panel 43S, of a controller 56 for operating the carriage portion 160. For example, an operation instruction including a moving instruction for the carriage portion 160 is input through the controller 56.

The controller 56 is provided with an auto button 56A, a return button 56C, and a direction key 56D. The auto button 56A is an operation button for inputting an automatic positioning instruction for automatically moving to the target position represented by the marker MR into the processor 40S. The return button 56C is an operation button for inputting a return instruction for returning the carriage portion 160 to, for example, a home position set in advance as an initial position of the carriage portion 160. The direction key 56D is an operation button for inputting a moving instruction for moving the carriage portion 160 in any direction of four directions.

The auto button 56A and the return button 56C are operation buttons for causing the carriage portion 160 to autonomously execute a traveling control set in advance. Meanwhile, the direction key 56D is an operation button for executing a simple traveling control of providing an instruction for only a direction and moving by an operation amount in the direction provided by the instruction. The direction key 56D is of a so-called dead man's type that continues outputting an operation signal during an operation and stops outputting the operation signal in a case where the operation stops. This is safer than a case where the operation signal is output even in a state where a hand is released from the direction key 56D.

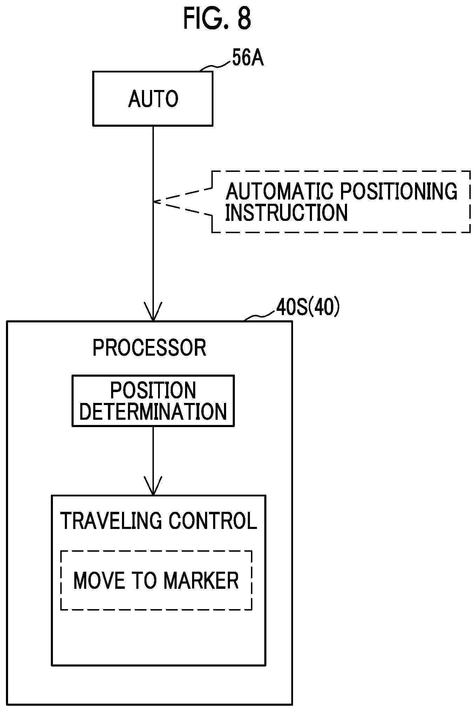

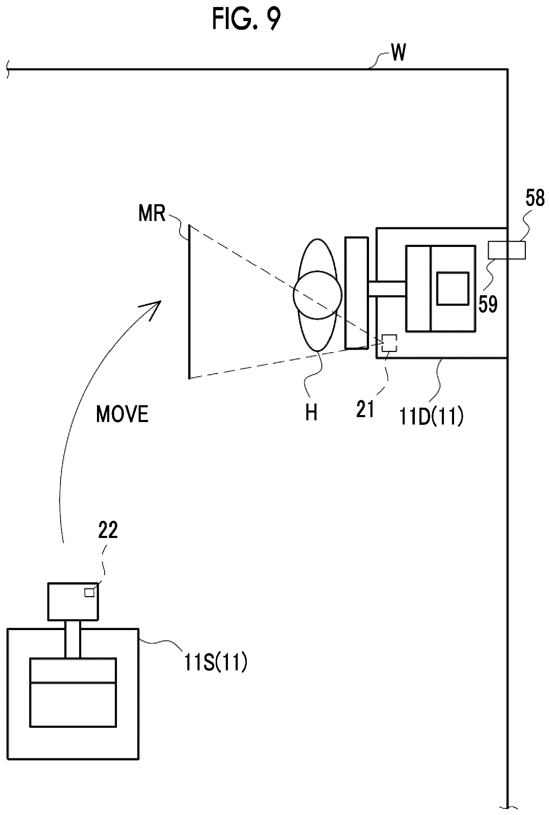

The traveling control of the carriage portion 160 executed by the processor 40S will be described with reference to Figs. 8 and 9. The autonomous traveling control executed by the carriage portion 160 is broadly divided into an automatic positioning control and the self position estimation. The automatic positioning control is a control of detecting the marker MR and moving the carriage portion 160 to the target position represented by the marker MR. For example, the example illustrated in Fig. 9 is an example of moving the radiation source unit 11S including the carriage portion 160 to the target position represented by the marker MR projected from the panel unit 11D after the panel unit 11D is positioned.

In the carriage portion 160, in a case where the automatic positioning instruction is input by operating the auto button 56A, the processor 40S starts detecting the marker MR. In detecting the marker MR, the processor 40S acquires the image 54 from the camera 22. For example, the processor 40S detects whether or not the marker MR is captured in the image 54 through image analysis. In a case where the marker MR is not present, the carriage portion 160 is caused to randomly travel to search for the marker MR. In performing the automatic positioning, the operator OP may move the radiation source unit 11S including the carriage portion 160 to a position near the marker MR. Doing so can reduce a time for searching for the marker MR.

In a case where the marker MR is detected based on the image 54, for example, the processor 40S, in the position determination, compares the reference position BP with the position of the marker MR in the image 54 and determines whether or not the marker MR is within the allowable range. In a case where the marker MR is not within the allowable range, the processor 40S moves the carriage portion 160 to the marker MR such that the marker MR falls within the allowable range. Accordingly, the radiation source unit 11S can be moved to the target position represented by the marker MR. For example, the automatic positioning is performed in this manner.

As illustrated in Fig. 9, the panel unit 11D including the projection apparatus 21 that projects the marker MR includes an engaging mechanism that engages with a location set in advance. For example, the location set in advance is a predetermined position in an imaging room. The engaging mechanism is an engaging portion 59 that mechanically engages with a pin 58 provided on a wall W of the imaging room. For example, the engaging portion 59 is a recessed portion that fits with a pin. During radiography, for example, the panel unit 11D is engaged at the location set in advance by the engaging mechanism, and the radiation source unit 11S is positioned with respect to the panel unit 11D in the engaged state.

In the self position estimation, the processor 40S acquires the measurement values from the measurement sensor MS and, for example, obtains the moving amount of the carriage portion 160 from the home position. For example, the home position is a position registered as a reference position of the radiation source unit 11S in the imaging room. As described above, the measurement sensor MS measures the measurement values such as the rotation amounts, the rotation directions, the revolution amounts, and the revolution angles of the wheels 16A. The processor 40S executes the self position estimation of estimating a relative self position of the carriage portion 160 with respect to the home position by accumulating the measured values. Such self position estimation is self position estimation based on a so-called odometry technique. By performing such self position estimation, the carriage portion 160 can be automatically returned to the home position.

In a case where the return button 56C of the controller 56 illustrated in Fig. 7 is operated, the processor 40S automatically returns the carriage portion 160 to the home position based on the self position estimation. A traveling control of automatically moving the carriage portion 160 to not only the home position but also a registered position set in advance can also be performed.

In the second embodiment, for example, the traveling mechanism is an electric traveling mechanism as illustrated by the carriage portion 160. Since electric traveling is enabled, movement of the imaging unit 11 is facilitated compared to that in manual traveling.

In the second embodiment, the radiation source unit 11S that is at least one imaging unit 11 (in the above example, the radiation source unit 11S) of the two imaging units 11 and that includes the optical sensor (for example, the camera 22) and the processor 40 (in the above example, the processor 40S) includes the electric traveling mechanism (for example, the carriage portion 160) that electrically travels on the floor. The processor 40S automatically performs positioning by controlling the carriage portion 160 based on the determination result related to the relative positional relationship. Thus, relative positioning between the imaging units 11 can be performed more simply than that in a case where the operator OP visually checks the marker MR and manually moves the imaging unit 11.

In the second embodiment, the panel unit 11D that is at least one imaging unit 11 (in the above example, the panel unit 11D) of the two imaging units 11 and that includes the projection apparatus 21 includes the engaging mechanism that engages with the location set in advance. The other imaging unit 11 (in the above example, the radiation source unit 11S) in which the processor 40S determines whether or not the relative positional relationship is appropriate based on the positioning indicator (for example, the marker MR) projected by the projection apparatus 21 includes the electric traveling mechanism (for example, the carriage portion 160) that electrically travels on the floor. The carriage portion 160 moves to the target position represented by the marker MR based on the determination result of the processor 40S. Since the imaging unit 11 including the projection apparatus 21 engages with the location set in advance, high accuracy of positioning is achieved. While the above example illustrates the engaging mechanism as an example of mechanical engaging, magnetic engaging using a magnet, an electromagnet, or the like may be adopted.

### Configuration Example of Two Imaging Units 11

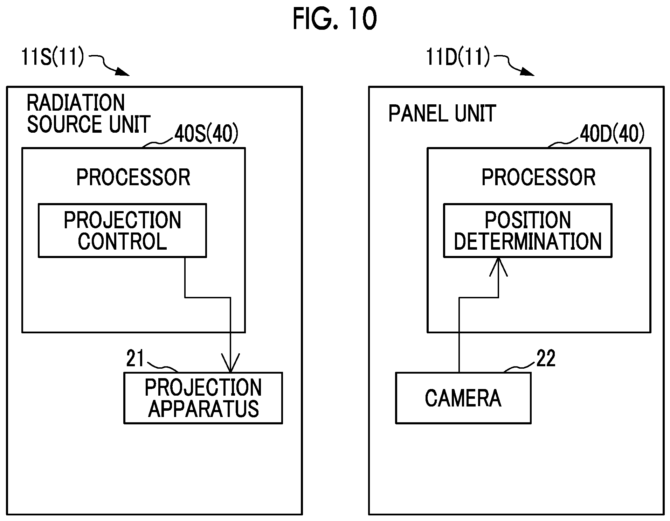

The first embodiment and the second embodiment describe an example of providing the projection apparatus 21 in the panel unit 11D out of the two imaging units 11 and providing the camera 22, which is an example of the optical sensor, and the processor 40S that performs the position determination based on the marker MR in the radiation source unit 11S. However, various modifications can be made to the configuration examples of the projection apparatus 21, the optical sensor, and the processor as illustrated in Figs. 10 to 14.

First, as illustrated in Fig. 10, contrary to each embodiment, the projection apparatus 21 may be provided in the radiation source unit 11S, and the camera 22 and a processor 40D that performs the position determination based on the marker MR may be provided in the panel unit 11D.

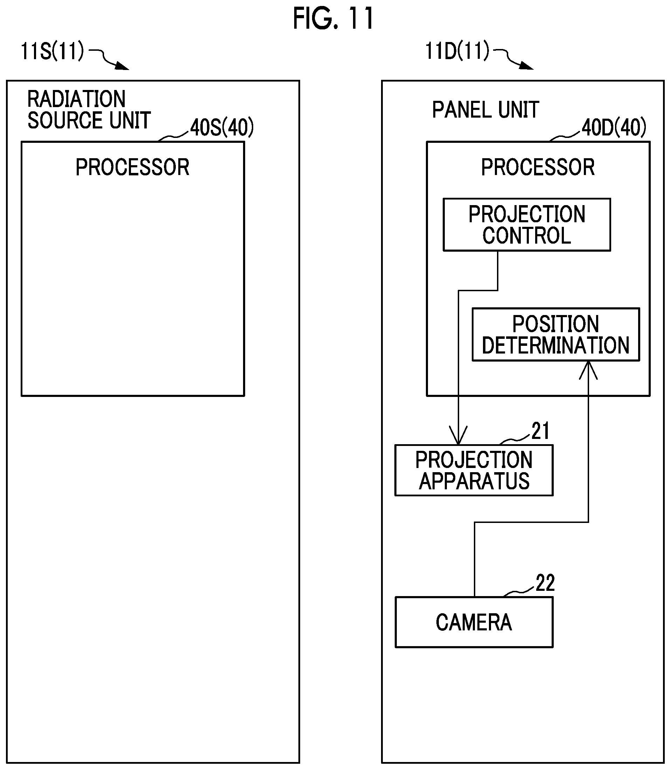

As illustrated in Fig. 11, one imaging unit 11 out of the two imaging units 11 may include the optical sensor and the processor 40 in addition to the projection apparatus 21. In the example illustrated in Fig. 11, the panel unit 11D includes the camera 22 and the processor 40D that performs the position determination based on the marker MR, in addition to the projection apparatus 21. In this case, the processor 40D of the panel unit 11D, which is the one imaging unit 11, determines whether or not the relative positional relationship is appropriate by determining whether or not the counterpart radiation source unit 11S, which is the other imaging unit 11, is present at the target position represented by the marker MR that is the positioning indicator projected by the projection apparatus 21 of the host panel unit 11D, which is the one imaging unit 11, and that is detected by the camera 22.

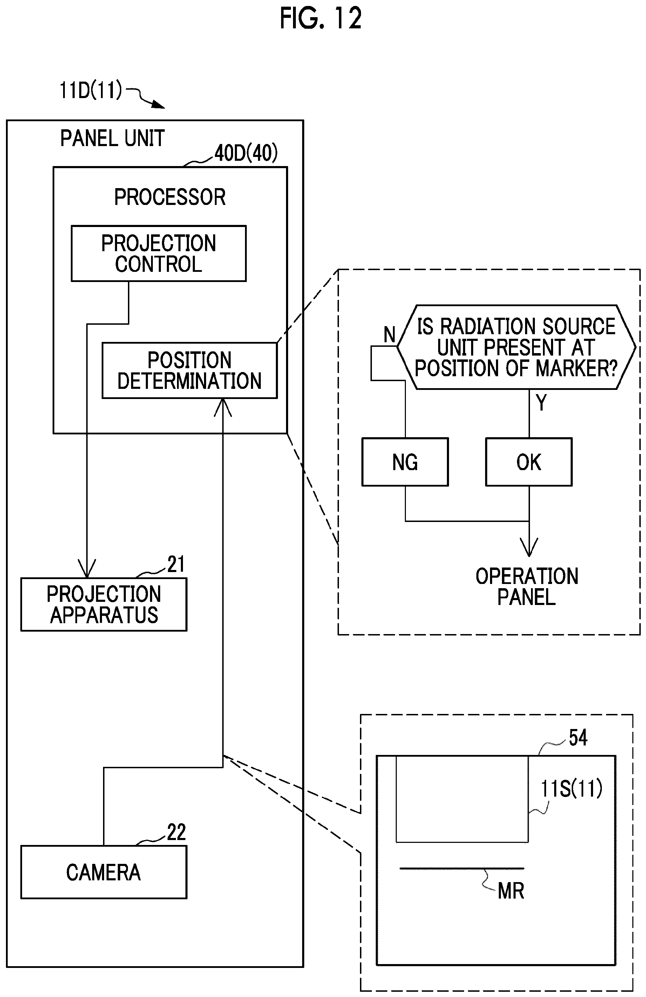

Specifically, as illustrated in Fig. 12, the processor 40D of the panel unit 11D acquires the image 54 from the camera 22 and, in the position determination, obtains the positional relationship between the marker MR and the radiation source unit 11S, which is the counterpart imaging unit 11, included in the image 54 through image analysis. For example, the radiation source unit 11S in the image 54 is detected by performing image recognition of the carriage portion 16 in the image 54 using a pattern matching technique. Then, for example, the processor 40D determines whether or not the radiation source unit 11S represented by the carriage portion 16 is present at the position of the marker MR in the image 54. In a case where the radiation source unit 11S is at the position of the marker MR, the processor 40D determines that the relative positional relationship between the panel unit 11D and the radiation source unit 11S is appropriate (indicated by OK). Meanwhile, in a case where the radiation source unit 11S is not present at the position of the marker MR, it is determined that the relative positional relationship between the panel unit 11D and the radiation source unit 11S is inappropriate (indicated by NG). As described in the first embodiment, determination of the appropriateness is performed based on whether or not the positions of the marker MR and the radiation source unit 11S are within an allowable range set in advance.

In a case where the camera 22 and the processor 40 that performs the position determination based on the marker MR are provided in one of the two imaging units 11 in addition to the projection apparatus 21, for example, an advantage of being able to use an imaging unit 11 of the related art not provided with the projection apparatus 21 and the like as the other imaging unit 11 is achieved.

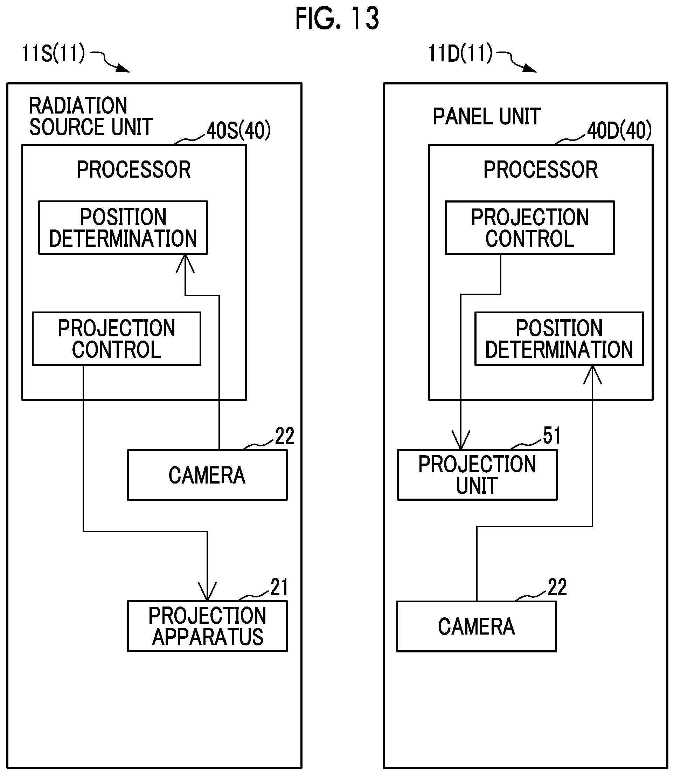

As illustrated in Fig. 13, each of the two imaging units 11 may include the camera 22 and the processor 40 that performs the position determination, in addition to the projection apparatus 21. By doing so, for example, which imaging unit 11 is to project the marker MR can be selected in accordance with a situation.

In this case, each processor 40 may determine the position of the host imaging unit 11 with respect to the marker MR projected by the counterpart imaging unit 11, as in the first embodiment. For example, this corresponds to a pattern in which the processor 40S of the radiation source unit 11S determines the position of the host radiation source unit 11S with respect to the marker MR projected by the counterpart panel unit 11D. Alternatively, as illustrated in Fig. 12, each processor 40 may determine the position of the counterpart imaging unit 11 with respect to the marker MR projected by the host imaging unit 11. For example, this corresponds to a pattern in which the processor 40D of the panel unit 11D determines the position of the counterpart radiation source unit 11S with respect to the marker MR projected by the host panel unit 11D.

In the example illustrated in Fig. 13, each processor 40 may perform the position determination of any of the patterns. The two patterns may be selectable in accordance with a situation.

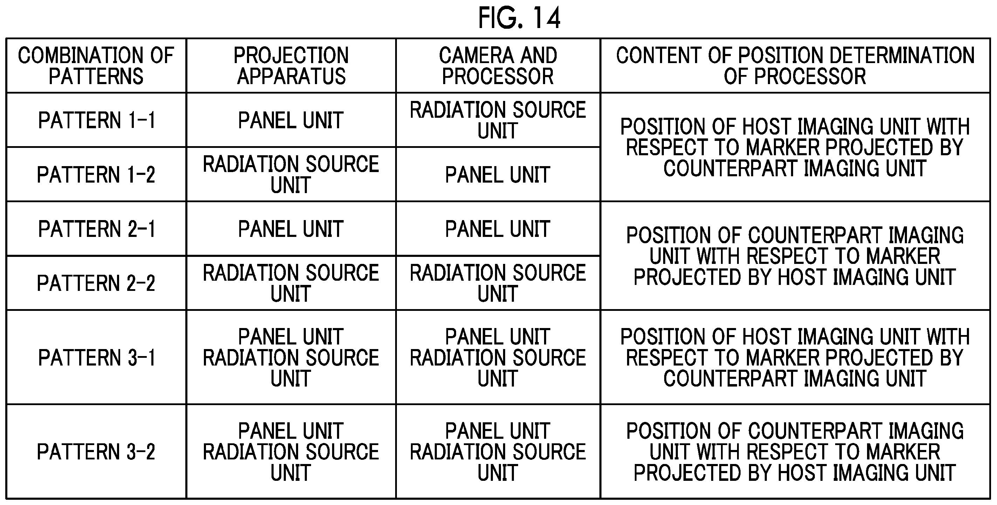

Fig. 14 illustrates a summary of the above. The table illustrated in Fig. 14 shows whether the projection apparatus 21, and the camera 22 and the processor 40 are provided in the panel unit 11D or the radiation source unit 11S, and a combination of content of the position determination of the processor 40.

Pattern 1-1 corresponds to the first embodiment, and Pattern 1-2 is a modification of Pattern 1-1 and is a pattern in which the radiation source unit 11S and the panel unit 11D are replaced with each other. Pattern 2-1 is a pattern in which the projection apparatus 21, the camera 22, and the like are provided in the panel unit 11D illustrated in Figs. 11 and 12, and Pattern 2-2 is a modification of Pattern 2-1 and is a pattern in which the projection apparatus 21, the camera 22, and the like are provided in the radiation source unit 11S. Patterns 3-1 and 3-2 are patterns in which the projection apparatus 21, the camera 22, and the like illustrated in Fig. 13 are provided in each of the radiation source unit 11S and the panel unit 11D. Pattern 3-1 has the same content of the position determination as Patterns 1-1 and 1-2, and Pattern 3-2 has the same content of the position determination as Patterns 2-1 and 2-2. Accordingly, various modifications can be made to the combination of the patterns.

Among those various patterns, Pattern 1-1 corresponding to the first embodiment achieves the following advantages. In Pattern 1-1, the radiation source unit 11S comprises the camera 22. Since the camera 22 has a role of detecting the marker MR from the captured image 54, it is not preferable that the subject H causing noise enters the imaging range SR. The subject H is disposed on the panel unit 11D side. Thus, in a case where the camera 22 is present on the radiation source unit 11S side, the subject H is less likely to enter the imaging range SR. In that sense, the radiation source unit 11S preferably comprises the camera 22.

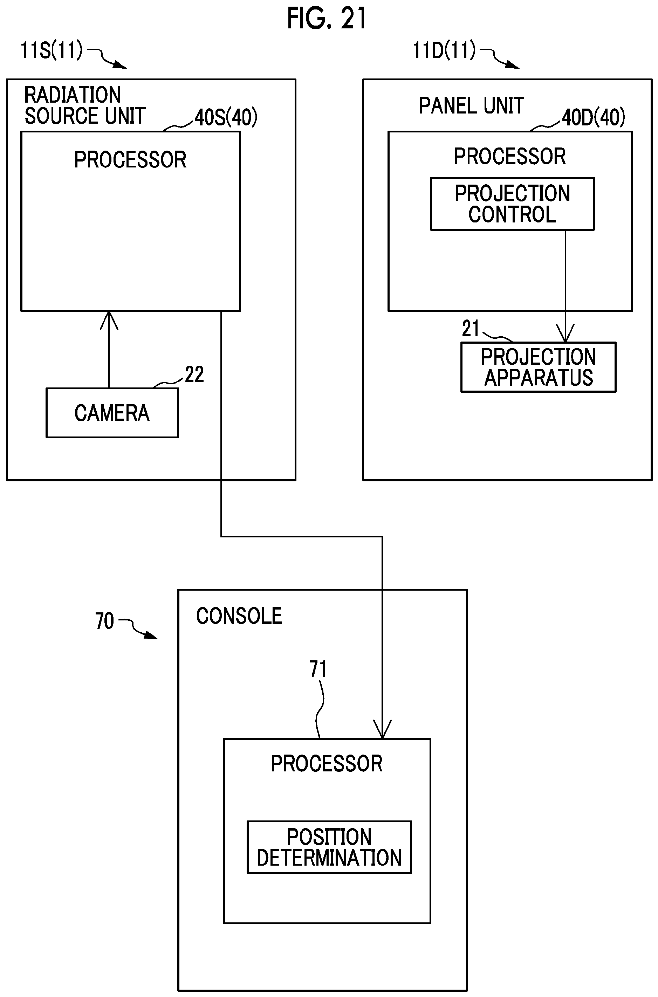

In all of the patterns illustrated in Fig. 14, the camera 22 and the processor 40 are provided in the same imaging unit 11. As will be described later with reference to Fig. 21, the processor that performs the position determination based on the image 54 may be provided in an apparatus different from the imaging unit 11. However, providing the camera 22 and the processor 40 in the same imaging unit 11 achieves the following advantages. First, in a case where the camera 22 and the processor 40 are provided in one imaging unit 11, a transmission time of the image 54 from the camera 22 to the processor 40 is shorter than that in a case where the camera 22 and the processor 40 are provided in different apparatuses. Thus, a processing speed is improved. An apparatus configuration can be simplified compared to that in a case where the camera 22 and the processor 40 are provided in different apparatuses.

As described in the first embodiment corresponding to Pattern 1-1 in Fig. 14, in a procedure of positioning, the radiation source unit 11S is generally moved in accordance with the position of the panel unit 11D after positioning the panel unit 11D in which the subject H is disposed. In this case, movement of the radiation source unit 11S is facilitated in a case where the determination result of the position determination is displayed on the operation panel 43S of the radiation source unit 11S. Thus, in a case where the imaging unit 11 in which the camera 22 and the processor 40 are provided is the radiation source unit 11S, the processor 40S of the radiation source unit 11S in which the camera 22 is provided can perform a series of processing such as acquisition of the image 54 from the camera 22, the position determination based on the image 54, and display of the determination result. Thus, processing efficiency is higher than that in a case where the camera 22 and the processor 40 are provided in different apparatuses.

In a case where the imaging unit 11 in which the camera 22 and the processor 40 are provided is the panel unit 11D as in Pattern 1-2 in Fig. 14, the following advantages are achieved. That is, the X-ray image after imaging is often displayed on the operation panel 43D of the panel unit 11D. In a case where the operator OP determines that positioning is inappropriate as a result of checking the X-ray image displayed on the operation panel 43D, the operator OP may position each imaging unit 11 again and perform reimaging. In this case, in a case where the position determination based on the image 54 from the camera 22 and the determination result are displayed on the operation panel 43D of the panel unit 11D, the captured X-ray image displayed on the same operation panel 43D can be referred to in performing positioning for reimaging. Accordingly, positioning for reimaging may be facilitated.

### Modification Example 1

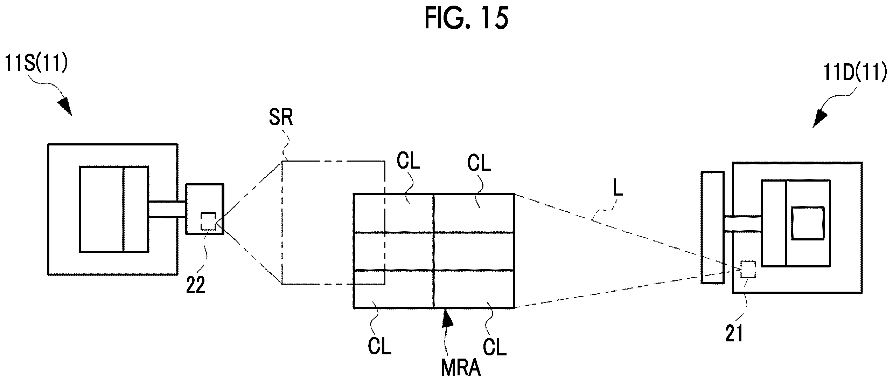

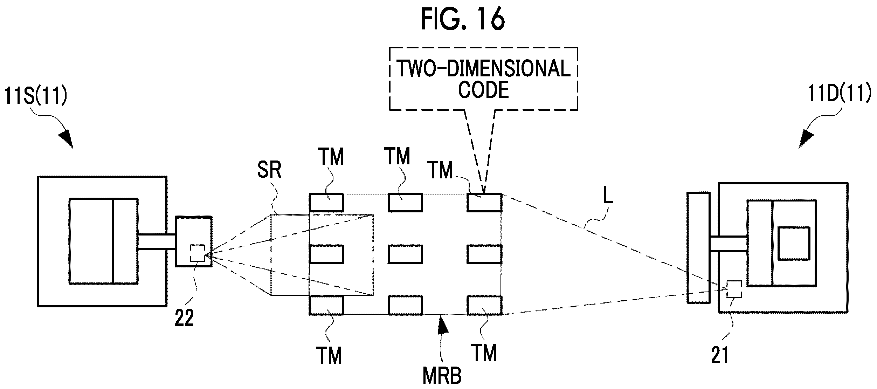

Other various modifications can be made to the radiography system 10. First, while the above embodiments describe the line-shaped marker MR as an example of the positioning indicator, the positioning indicator is not limited to the line-shaped marker MR. For example, a positioning indicator (for example, indicated by a marker MRA or a marker MRB) as in Modification Example 1 illustrated in Figs. 15 and 16 may be adopted. Modification Example 1 is an example of projecting a positioning indicator representing a plurality of target positions as the positioning indicator projected by the projection apparatus 21.

The marker MRA illustrated in Fig. 15 is a positioning indicator composed of a lattice pattern. For example, each of a plurality of cells CL divided by the lattice represents the target position. For example, lines dividing each cell CL have different colors, thicknesses, and the like, and because of these differences, the processor 40 can identify each cell CL of the marker MRA from the image 54 acquired from the camera 22. The processor 40 can perform the position determination for the target position corresponding to the designated imaging menu by associating the imaging menu with each cell CL.

For example, in a case where the operator OP manually positions the imaging unit 11, an audio message such as "Current position corresponds to a certain imaging menu" may be output in a state where the imaging unit 11 is aligned with any of the plurality of target positions. In a case where such an audio message is provided, the operator OP can easily obtain the current position of the imaging unit 11. A similar text message instead of the audio message may be output to the operation panel 43.

In a case where the imaging unit 11 is positioned at the target position represented by a certain cell CL, fine adjustment such as moving by 10 cm in a lateral direction from the position may be performed. In this case, for example, fine adjustment such as moving the adjacent cell CL as the target position can be performed in a case where a spacing of the lattice of the marker MRA is set to a 10 cm pitch.

While the plurality of cells CL are illustrated as the target positions with respect to the lattice marker MRA, each of the plurality of lines constituting the lattice may be used as the target position.

The marker MRB illustrated in Fig. 16 is a positioning indicator having a pattern image in which a plurality of target positions TM are arranged. For example, the target positions TM are two-dimensional codes. For example, two-dimensional barcodes such as QR codes (registered trademark) are used as the two-dimensional codes. As in the case of the marker MRA illustrated in Fig. 15, the processor 40 can identify each target position TM of the marker MRB from the image 54 acquired from the camera 22. The processor 40 can perform the position determination for the target position corresponding to the imaging menu by associating the imaging menu with each target position TM.

In the marker MRB, the two-dimensional codes may be codes other than two-dimensional barcodes. For example, two-dimensional codes that are arrangement patterns of a plurality of dots and in which each arrangement pattern can be identified by setting a unique size and a unique position for each dot may be adopted. For example, such two-dimensional codes are known as Anoto (registered trademark) patterns.

In a case where the projection apparatus 21 projects the positioning indicator representing the plurality of target positions as the positioning indicator, a wider variety of use than that of the positioning indicator representing a single target position is achieved.

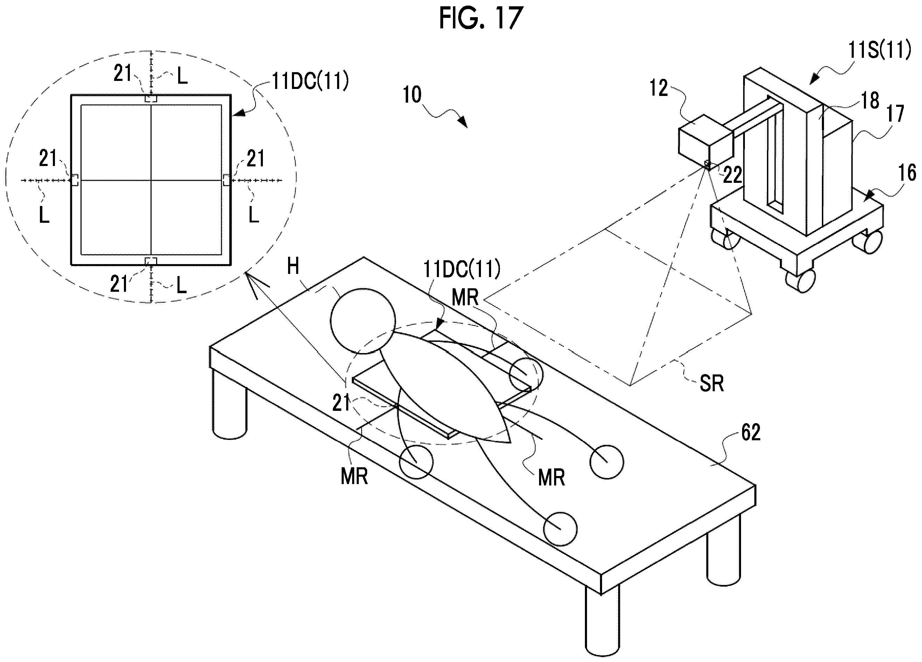

While each embodiment describes an example of the panel unit 11D including the traveling mechanism such as the carriage portion 16, the panel unit 11D may not include the traveling mechanism. A portable panel unit 11DC illustrated in Fig. 17 is a radiation detection panel having a portable housing. The housing has a flat plate shape having a rectangular planar shape. This panel unit 11DC is called an electronic cassette or the like. As illustrated in Fig. 17, for example, the panel unit 11DC is used for imaging the subject H in a decubitus posture on a decubitus imaging table 62. The panel unit 11DC is interposed between a top plate of the decubitus imaging table 62 and the subject H. In this state, the emission portion 12 of the radiation source unit 11S is disposed above the subject H, and imaging is performed. In such imaging, most of the panel unit 11DC is covered by the subject H. Thus, it is difficult to check a center of the panel unit 11DC.

The panel unit 11DC illustrated in Fig. 17 is provided with the projection apparatus 21 on each side of the rectangular housing. For example, the projection apparatus 21 is a line laser projector using an LD as a light source. The projection apparatus 21 provided on each side of the housing projects the line-shaped marker MR to the outside of the housing as the positioning indicator. The projection apparatus 21 emits a line-shaped beam L from the LD. The beam L is projected to the top plate of the decubitus imaging table 62 and is visible as the marker MR.

The camera 22 of the radiation source unit 11S acquires the image 54 including the marker MR, and the processor 40S performs the position determination based on the marker MR. The position determination may be performed by the operator OP instead of the processor 40S. That is, the operator OP may visually check the marker MR, estimate a center position of the panel unit 11DC behind the subject H, and position the emission portion 12 of the radiation source unit 11S with respect to the panel unit 11DC. By projecting the line-shaped marker MR via the panel unit 11DC, positioning in using the portable panel unit 11DC can be performed more simply than that in the related art.

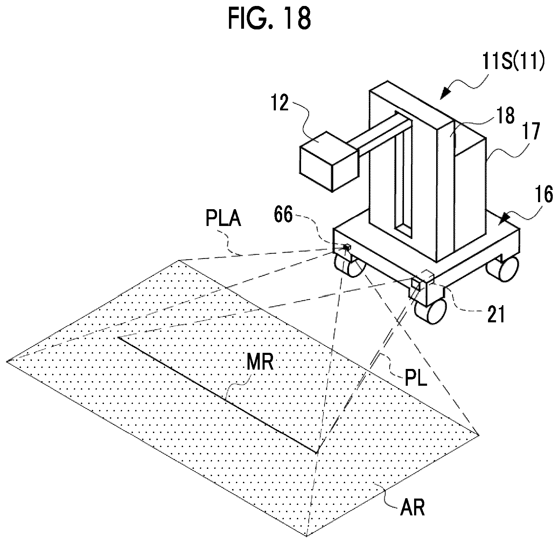

As illustrated in Fig. 18, at least one imaging unit 11 of the two imaging units 11 may include a projection apparatus that projects a region indicator indicating a region requiring entry with caution in addition to the positioning indicator. In the example illustrated in Fig. 18, the radiation source unit 11S is provided with a projection apparatus 66 in addition to the projection apparatus 21. The projection apparatus 66 projects a region indicator AR to the floor by emitting projection light PLA. For example, the region indicator AR is a region indicator indicating a region in which the counterpart imaging unit 11 of positioning enters or exposure to the X-rays may occur and that requires entry with caution for the operator OP. Projecting the region indicator AR can cause the operator OP to exercise caution. While the projection apparatus 66 is provided separately from the projection apparatus 21, functions of the projection apparatus 66 may be implemented by the projection apparatus 21.

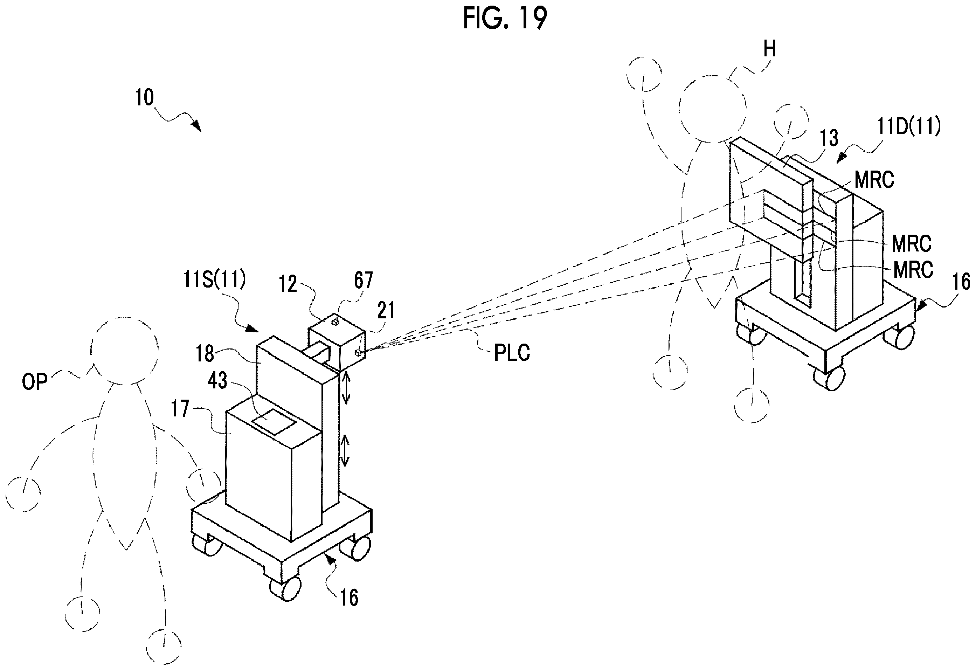

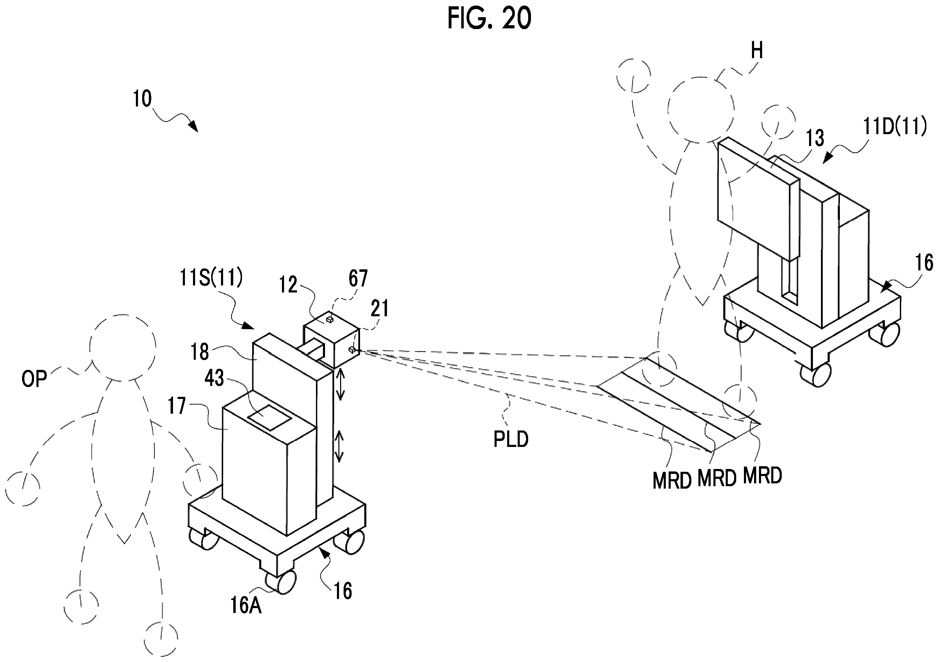

As illustrated in Figs. 19 and 20, at least one imaging unit 11 of the two imaging units 11 may project a marker for a different purpose, such as a marker MRC and a marker MRD, in addition to the positioning indicator. For example, the marker MRC and the marker MRD are projected by emitting projection light PLC and PLD via the projection apparatus 21. While the projection apparatus 21 projects the marker MRC and the marker MRD in the examples illustrated in Figs. 19 and 20, a projection apparatus different from the projection apparatus 21 may be used.

The imaging unit 11 illustrated in Figs. 19 and 20 is, for example, the radiation source unit 11S. For example, the radiation source unit 11S is provided with a measurement apparatus 67 that measures dimensions such as a height and a body thickness of the subject H. For example, the measurement apparatus 67 measures the height by acquiring an image of the subject H or measures the body thickness of the subject H using a distance-measuring sensor. The marker MRC and the marker MRD are used for calibrating the measurement apparatus 67 or correcting measured values. For example, the marker MRC illustrated in Fig. 19 is an indicator projected to a plurality of known heights set in advance. For example, the measurement apparatus 67 calibrates the measured values by measuring a height of each marker MRC. The marker MRD illustrated in Fig. 20 is an indicator projected to a plurality of known positions set in advance in a thickness direction of the subject H. For example, the measurement apparatus 67 corrects the measurement value of the body thickness of the subject H with reference to a position of each marker MRD.

In all of the patterns illustrated in Fig. 14, the camera 22 and the processor 40 that determines whether or not the relative positional relationship between the two imaging units 11 is appropriate based on the image 54 including the marker MR are provided in the imaging unit 11. However, as illustrated in Fig. 21, an apparatus different from the two imaging units 11 may comprise a processor 71 that performs the position determination. For example, the different apparatus is a console 70. The console 70 is an apparatus for operating at least one of the two imaging units 11. In the example illustrated in Fig. 21, the radiation source unit 11S comprises the camera 22, and the console 70 comprises the processor 71. The console 70 acquires the image 54 from the camera 22 of the radiation source unit 11S, and the processor 71 performs the position determination based on the image 54 including the marker MR. The determination result may be displayed on a display of the console 70 or may be transmitted to the radiation source unit 11S and displayed on the operation panel 43S. The different apparatus may be an apparatus other than the console 70.

As illustrated in Fig. 17, in the radiography system according to the disclosed technology, the optical sensor and the processor are not essential, and at least one of the two imaging units may include the projection apparatus.

For the autonomous traveling control of the electric traveling mechanism illustrated by the carriage portion 160 as an example, a simultaneous localization and mapping (SLAM) technique for autonomously traveling while performing the self position estimation and map creation at the same time may be used.

In the traveling mechanism illustrated in the above embodiments, the form of the wheels is for illustrative purposes, and various modifications can be made. For example, the number of front wheels and/or the number of rear wheels may be changed independently of each other. For example, a combination of one front wheel and two rear wheels or a combination of two front wheels and three rear wheels may be adopted. For example, sizes and shapes may be changed between the front wheels and the rear wheels.

In the above embodiments, various types of hardware described below can be used as hardware of the processor 40. The various types of hardware include, in addition to a CPU that is a general-purpose processor functioning as various processing portions by executing software (a program), a programmable logic device (PLD) such as a field-programmable gate array (FPGA) that has a circuit configuration changeable after manufacture, a dedicated electric circuit such as an application specific integrated circuit (ASIC) that has a circuit configuration dedicatedly designed to execute specific processing, and the like.

The above various types of processing may be executed by one of the various types of hardware or may be executed by a combination of two or more pieces of hardware of the same type or different types (for example, a plurality of FPGAs and a combination of a CPU and an FPGA). A plurality of processing portions may be composed of one piece of hardware. Examples of the plurality of processing portions composed of one piece of hardware include a form of using hardware that implements functions of the whole system including the plurality of processing portions in one integrated circuit (IC) chip, like a system on chip (SOC).

The various processing portions are composed of one or more of the various types of hardware.

More specifically, electric circuits (circuitry) obtained by combining circuit elements such as semiconductor elements can be used as various hardware structures.

The disclosed technology also includes, in addition to a program for operating the imaging unit, a computer-readable storage medium (a universal serial bus (USB) memory, a digital versatile disc (DVD)-read only memory (ROM), or the like) that stores the program in a non-transitory manner. The disclosed technology can also be applied to a program and a program product.

Content of description and content of illustration shown above are detailed description for parts according to the disclosed technology and are merely an example of the disclosed technology. For example, description related to the above configurations, functions, actions, and effects is description related to examples of configurations, functions, actions, and effects of the parts according to the disclosed technology. Thus, unnecessary parts may be removed, new elements may be added, or the parts may be replaced with each other in the content of description and the content of illustration shown above without departing from the scope of the disclosed technology. Particularly, description related to common technical knowledge or the like not required to be described for embodying the disclosed technology is omitted in the content of description and the content of illustration shown above in order to avoid complication and facilitate understanding of the parts according to the disclosed technology.

The technology according to the following appendices can be perceived from the above description.
Appendix 1
   A radiography system used for radiography, comprising:
   two imaging units including an imaging unit including a radiation source and an imaging unit including a radiation detector,
   in which at least one imaging unit of the two imaging units includes a projection apparatus that projects a positioning indicator used for relative positioning between the two imaging units.
Appendix 2
   The radiography system according to Appendix 1,
   in which at least one imaging unit of the two imaging units includes an optical sensor that optically detects the positioning indicator, and
   a processor configured to determine whether or not a relative positional relationship between the two imaging units is appropriate based on the positioning indicator detected by the optical sensor is provided.
Appendix 3
   The radiography system according to Appendix 2,
   in which, in a case where at least one imaging unit of the two imaging units includes the projection apparatus and the other imaging unit includes the optical sensor and the processor,
   the processor included in the other imaging unit is configured to determine whether or not the relative positional relationship is appropriate by determining whether or not a host imaging unit which is the other imaging unit is present at a target position represented by the positioning indicator that is projected by the projection apparatus of a counterpart imaging unit which is the one imaging unit and that is detected by the optical sensor.
Appendix 4
   The radiography system according to Appendix 2 or 3,
   in which, in a case where at least one imaging unit of the two imaging units includes the optical sensor and the processor in addition to the projection apparatus,
   the processor included in the one imaging unit is configured to determine whether or not the relative positional relationship is appropriate by determining whether or not a counterpart imaging unit which is the other imaging unit is present at a target position represented by the positioning indicator that is projected by the projection apparatus of a host imaging unit which is the one imaging unit and that is detected by the optical sensor.
Appendix 5
   The radiography system according to any one of Appendices 2 to 4,
   in which each of the two imaging units includes the optical sensor and the processor in addition to the projection apparatus.
Appendix 6
   The radiography system according to any one of Appendices 2 to 4,
   in which at least the imaging unit including the radiation detector out of the two imaging units includes the projection apparatus.
Appendix 7
   The radiography system according to any one of Appendices 1 to 6,
   in which at least one imaging unit of the two imaging units includes a traveling mechanism that travels on a floor.
Appendix 8
   The radiography system according to Appendix 7,
   in which the traveling mechanism is an electric traveling mechanism.
Appendix 9
   The radiography system according to any one of Appendices 2 to 6,
   in which the imaging unit that is at least one of the two imaging units and that includes the optical sensor and the processor includes an electric traveling mechanism that electrically travels on a floor, and
   the processor is configured to automatically perform registration by controlling the electric traveling mechanism based on a determination result related to the relative positional relationship.
Appendix 10
   The radiography system according to any one of Appendices 2 to 6,
   in which the imaging unit that is at least one of the two imaging units and that includes the projection apparatus includes an engaging mechanism that engages with a location set in advance,
   the other imaging unit in which the processor is configured to determine whether or not the relative positional relationship is appropriate based on the positioning indicator projected by the projection apparatus includes an electric traveling mechanism that electrically travels on a floor, and
   the electric traveling mechanism moves to a target position represented by the positioning indicator based on a determination result of the processor.
Appendix 11
   The radiography system according to Appendix 2,
   in which the processor is provided in an apparatus different from the two imaging units.
Appendix 12
   The radiography system according to Appendix 11,
   in which the different apparatus is a console for operating at least one of the two imaging units.
Appendix 13
   The radiography system according to any one of Appendices 1 to 12,
   in which the projection apparatus determines a projection position of the positioning indicator in accordance with a designated imaging menu.
Appendix 14
   The radiography system according to any one of Appendices 1 to 13,
   in which the projection apparatus projects a positioning indicator representing a plurality of target positions as the positioning indicator.
Appendix 15
   The radiography system according to any one of Appendices 1 to 6,
   in which the imaging unit including the radiation detector is a radiation detection panel having a portable housing, and
   the projection apparatus projects a line-shaped marker to an outside of the housing as the positioning indicator.
Appendix 16
   The radiography system according to any one of Appendices 1 to 15,
   in which at least one imaging unit of the two imaging units includes a projection apparatus that projects a region indicator indicating a region requiring entry with caution in addition to the positioning indicator.
Appendix 17
   An imaging unit that is used for radiography and that is one of two imaging units including an imaging unit including a radiation source and an imaging unit including a radiation detector, the imaging unit comprising:
   a projection apparatus that projects a positioning indicator used for relative positioning between the two imaging units.

In the present specification, "A and/or B" is synonymous with "at least one of A or B". That is, "A and/or B" may mean only A, only B, or a combination of A and B. In the present specification, the same approach as "A and/or B" also applies to an expression of three or more matters connected with "and/or".

All documents, patent applications, and technical standards described in the present specification are incorporated in the present specification by reference to the same extent as those in a case where individual documents, patent applications, and technical standards are specifically and individually indicated to be incorporated by reference.

## Claims

1. A radiography system (10) used for radiography, comprising:
two imaging units (11) including an imaging unit (11S) including a radiation source (12) and an imaging unit (11D) including a radiation detector (13),
wherein at least one imaging unit of the two imaging units (11) includes a projection apparatus (21) that projects a positioning indicator used for relative positioning between the two imaging units (11).

2. The radiography system (10) according to claim 1,
wherein at least one imaging unit of the two imaging units (11) includes an optical sensor (22) that optically detects the positioning indicator, and
a processor configured to determine whether or not a relative positional relationship between the two imaging units (11) is appropriate based on the positioning indicator detected by the optical sensor (22) is provided.

3. The radiography system (10) according to claim 2,
wherein, in a case where at least one imaging unit of the two imaging units (11) includes the projection apparatus (21) and the other imaging unit includes the optical sensor (22) and the processor,
the processor included in the other imaging unit is configured to determine whether or not the relative positional relationship is appropriate by determining whether or not a host imaging unit which is the other imaging unit is present at a target position represented by the positioning indicator that is projected by the projection apparatus (21) of a counterpart imaging unit which is the one imaging unit and that is detected by the optical sensor (22).

4. The radiography system (10) according to claim 2,
wherein, in a case where at least one imaging unit of the two imaging units (11) includes the optical sensor (22) and the processor in addition to the projection apparatus (21),
the processor included in the one imaging unit is configured to determine whether or not the relative positional relationship is appropriate by determining whether or not a counterpart imaging unit which is the other imaging unit is present at a target position represented by the positioning indicator that is projected by the projection apparatus (21) of a host imaging unit which is the one imaging unit and that is detected by the optical sensor (22).

5. The radiography system (10) according to claim 2,
wherein each of the two imaging units (11) includes the optical sensor (22) and the processor in addition to the projection apparatus (21).

6. The radiography system (10) according to claim 1,
wherein at least the imaging unit (11D) including the radiation detector (13) out of the two imaging units (11) includes the projection apparatus (21).

7. The radiography system (10) according to claim 1,
wherein at least one imaging unit of the two imaging units (11) includes a traveling mechanism (16, 160) that travels on a floor.

8. The radiography system (10) according to claim 7,
wherein the traveling mechanism is an electric traveling mechanism (160).

9. The radiography system (10) according to claim 2,
wherein the imaging unit that is at least one of the two imaging units (11) and that includes the optical sensor (22) and the processor includes an electric traveling mechanism (160) that electrically travels on a floor, and
the processor is configured to automatically perform registration by controlling the electric traveling mechanism (160) based on a determination result related to the relative positional relationship.

10. The radiography system (10) according to claim 2,
wherein the imaging unit that is at least one of the two imaging units (11) and that includes the projection apparatus (21) includes an engaging mechanism that engages with a location set in advance,
the other imaging unit in which the processor is configured to determine whether or not the relative positional relationship is appropriate based on the positioning indicator projected by the projection apparatus (21) includes an electric traveling mechanism (160) that electrically travels on a floor, and
the electric traveling mechanism (160) moves to a target position represented by the positioning indicator based on a determination result of the processor.

11. The radiography system (10) according to claim 2,
wherein the processor is provided in an apparatus different from the two imaging units (11).

12. The radiography system (10) according to claim 11,
wherein the different apparatus is a console (70) for operating at least one of the two imaging units (11).

13. The radiography system (10) according to claim 1,
wherein the projection apparatus (21) determines a projection position of the positioning indicator in accordance with a designated imaging menu.

14. The radiography system (10) according to claim 1,
wherein the projection apparatus (21) projects a positioning indicator representing a plurality of target positions as the positioning indicator.

15. The radiography system (10) according to claim 1,
wherein the imaging unit (11D) including the radiation detector (13) is a radiation detection panel having a portable housing, and
the projection apparatus (21) projects a line-shaped marker to an outside of the housing as the positioning indicator.

16. The radiography system (10) according to claim 1,
wherein at least one imaging unit of the two imaging units (11) includes a projection apparatus (21) that projects a region indicator indicating a region requiring entry with caution in addition to the positioning indicator.

17. An imaging unit that is used for radiography and that is one of two imaging units (11) including an imaging unit (11S) including a radiation source (12) and an imaging unit (!!D) including a radiation detector (13), the imaging unit comprising:
a projection apparatus (21) that projects a positioning indicator used for relative positioning between the two imaging units (11).
